(19) Europäisches Patentamt
European Patent Office
Office européen des brevets

(11) Veröffentlichungsnummer: **0 231 152 A2**

# (12) EUROPÄISCHE PATENTANMELDUNG

(21) Anmeldenummer: **87810047.8**

(22) Anmeldetag: **26.01.87**

(51) Int. Cl.[4]: **C 07 C 127/22**
C 07 C 93/14,
C 07 C 119/048, A 01 N 47/34

(30) Priorität: **30.01.86 CH 345/86 05.12.86 CH 4863/86**

(43) Veröffentlichungstag der Anmeldung:
**05.08.87 Patentblatt 87/32**

(84) Benannte Vertragsstaaten:
**AT BE CH DE ES FR GB IT LI NL**

(71) Anmelder: **CIBA-GEIGY AG**
**Klybeckstrasse 141**
**CH-4002 Basel (CH)**

(72) Erfinder: **Ehrenfreund, Josef, Dr.**
**Amselstrasse 11**
**CH-4123 Allschwil (CH)**

(54) **Phenylbenzoylharnstoffe.**

(57) Neue substituierte N-[4-(Halogenalkoxy)-trifluormethyl-phenyl]-N'-benzoylharnstoffe der Formel

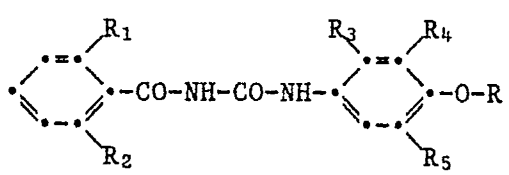

,

worin
R $C_1$-$C_4$-Halogenalkyl mit 1 bis 9 Halogenatomen;
$R_1$ Wasserstoff, Halogen, Methyl, Aethyl, Methoxy oder Aethoxy:
$R_2$ Halogen oder Methoxy;
$R_3$ Halogen oder Trifluormethyl; und
$R_4$ und $R_5$ unabhängig voneinander Wasserstoff, Halogen oder Trifluormethyl bedeuten, mit der Massgabe, dass mindestens einer der Reste $R_3$, $R_4$ und $R_5$ die Bedeutung Trifluormethyl besitzt, und dass $R_4$ und $R_5$ nicht gleichzeitig Wasserstoff bedeuten;
Verfahren und Zwischenprodukte zur Herstellung dieser Verbindungen sowie diese enthaltende Mittel zur Verwendung in die Schädlingsbekämpfung, insbesondere zur Bekämpfung von Pflanzen und Tiere befallenden Insekten und Vertretern der Ordnung Akarina. Die neuen Verbindungen weisen insbesondere larvizide Wirksamkeit gegen pflanzenschädigende Insekten auf.

EP 0 231 152 A2

**Beschreibung**

## Phenylbenzolharnstoffe

Die vorliegende Erfindung betrifft neue substituierte N-[4-(Halogenalkyloxy)-trifluormethyl-phenyl]-N'-benzoylharnstoffe, Verfahren zu ihrer Herstellung und ihre Verwendung in der Schädlingsbekämpfung.

Diese erfindungsgemässen Verbindungen haben die Formel I

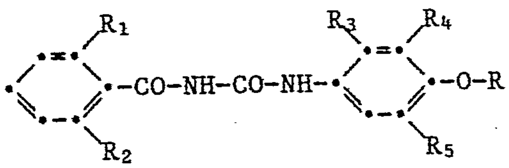
(I),

worin
R $C_1$-$C_4$-Halogenalkyl mit 1 bis 9 Halogenatomen;
$R_1$ Wasserstoff, Halogen, Methyl, Aethyl, Methoxy oder Aethoxy;
$R_2$ Halogen oder Methoxy;
$R_3$ Halogen oder Trifluormethyl; und
$R_4$ und $R_5$ unabhängig voneinander Wasserstoff, Halogen oder Trifluormethyl bedeuten, mit der Massgabe, dass mindestens einer der Reste $R_3$, $R_4$ und $R_5$ die Bedeutung Trifluormethyl besitzt und dass $R_4$ und $R_5$ nicht gleichzeitig Wasserstoff bedeuten.

Unter Halogen sind im Rahmen der vorliegenden Erfindung bevorzugt Fluor, Chlor und Brom zu verstehen, insbesondere Fluor und Chlor.

Wegen ihrer Wirkung als Schädlingsbekämpfungsmittel bevorzugt sind Verbindungen der Formel I, die dadurch gekennzeichnet sind, dass R mit 1 bis 7 Halogenatomen substituiertes $C_1$-$C_3$-Alkyl oder mit 1 bis 9 Halogenatomen substituiertes $C_2$-$C_4$-Alkyl bedeutet.

Hervorzuheben sind insbesondere auch diejenigen Verbindungen der Formel I, worin $R_3$ Chlor oder Trifluormethyl, $R_4$ und $R_5$ unabhängig voneinander Wasserstoff, Chlor oder Trifluormethyl bedeuten; diejenigen Verbindung der Formel I, worin $R_1$ Wasserstoff, Fluor oder Chlor und $R_2$ Fluor oder Chlor bedeuten; sowie diejenigen Verbindungen der Formel I worin $R_1$ und $R_2$ unabhängig voneinander Fluor oder Chlor oder gleichzeitig Fluor bedeuten.

Besondere Erwähnung verdienen daneben solche Verbindungen der Formel I, worin R einen der Reste $-CHF_2$, $-CF_3$, $-CF_2CHF_2$, $-CH_2-CF_3$, $-CF_2CF_3$, $-CF_2CHFCl$, $-CF_2CHCl_2$, $-CF_2CCl_3$, $-CF_2CHBr_2$, $-CF_2CHFBr$, $-CF_2CHBr_2$,$-CH_2CHBrCH_2Br$ oder $-CF_2CHFCF_3$, vorzugsweise $-CF_2CHF_2$, $-CF_2CHFCl$, $-CF_2CHFBr$ oder $-CF_2CHFCF_3$, und speziell den Rest $-CF_2CHFCF_3$ bedeutet.

Die Verbindungen der Formel I können nach an sich bekannten Verfahren hergestellt werden (vgl. u.a. die deutschen Offenlegungsschriften Nr. 2.123.236, 2.601.780 und die europäische Patentanmeldung 13 414).

So kann man z.B. eine Verbindung der Formel I dadurch erhalten, dass man

a) eine Verbindung der Formel II

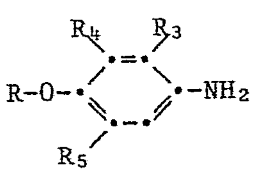
(II)

mit einer Verbindung der Formel III

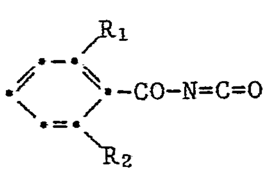
(III)

oder

b) eine Verbindung der Formel IV

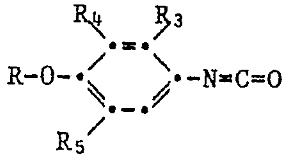

(IV)

mit einer Verbindung der Formel V

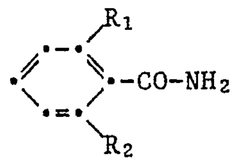

(V),

oder

c) eine Verbindung der Formel II mit einer Verbindung der Formel VI

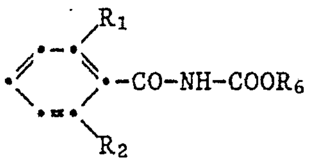

(VI)

umsetzt.

In den obigen Formeln II, III, IV, V und VI haben die Reste R, $R_1$, $R_2$, $R_3$, $R_4$ und $R_5$ die unter Formel I vorstehend angegebenen Bedeutungen und $R_6$ bedeutet einen gegebenenfalls mit Halogen, vorzugsweise Chlor, substituierten $C_1$-$C_8$-Alkylrest.

Die erwähnten Verfahren a) bis c) können vorzugsweise unter normalem Druck und in Gegenwart eines organischen Lösungs- oder Verdünnungsmittels durchgeführt werden. Als Lösungs- oder Verdünnungsmittel eignen sich z.B. Aether und ätherartige Verbindungen, wie Diäthyläther, Dipropyläther, Dibutyläther, Dioxan, Dimethoxyäthan und Tetrahydrofuran; N,N-dialkylierte Carbonsäureamide; aliphatische, aromatische sowie halogenierte Kohlenwasserstoffe, insbesondere Benzol, Toluol, Xylol, Chloroform, Methylenchlorid, Tetrachlor kohlenstoff und Chlorbenzol; Nitrile, wie Acetonitril oder Propionitril; Dimethylsulfoxid sowie Ketone, z.B. Aceton, Methyläthylketon, Methylisopropylketon und Methylisobutylketon. Verfahren a) wird im allgemeinen bei einer Temperatur von -10 bis 100° C, vorzugsweise zwischen 15 und 25° C, gegebenenfalls in Gegenwart einer organischen Base, z.B. Triäthylamin, durchgeführt. Die Durchführung von Verfahren b) erfolgt bei einer Temperatur von 0 bis 150° C, vorzugsweise beim Siedepunkt des verwendeten Lösungsmittels, und gegebenenfalls in Gegenwart einer organischen Base, wie Pyridin, und/oder unter Zusatz eines Alkali- oder Erdalkalimetalls, vorzugsweise Natrium. Für die Umsetzung der Urethane VI gemäss Verfahren c) wählt man im allgemeinen Temperaturen zwischen 60° C und dem Siedepunkt des Reaktionsgemisches. Als Lösungsmittel eignen sich vor allem aromatische Kohlenwasserstoffe, wie Toluol, Xylol, Chlorbenzol usw.

Die Ausgangsstoffe der vorstehenden Formeln II, III, IV, V und VI sind bekannt oder lassen sich, falls sie neu sind, analog bekannten Verfahren herstellen. Bei den substituierten Anilinen der Formel II handelt es sich um neue Verbindungen, die ebenfalls einen Gegenstand der vorliegenden Erfindung bilden und gemäss literaturbekannten Verfahren herstellbar sind, indem man z.B. entsprechend substituierte 4-Acetaminophenole mit Hexafluorpropylen analog der in J. Am. Chem. Soc. 73 (1951), 5831, beschriebenen Arbeitsweise veräthert. Anschliessend wird in dem gebildeten 4-Acetamino-(halogenalkyl)-phenyläther die N-Acetylgruppe in üblicher Weise abgespalten, um zu dem Anilin der Formel II zu gelangen. Weiterhin sind bestimmte substituierte Aniline der Formel II, worin $R_3$ Halogen bedeutet, durch direkte Halogenierung von Anilinen der Formel VII

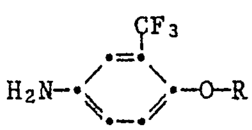

(VII),

worin R die vorstehend angegebene Bedeutung hat, zugänglich (vgl. Synthesis 1985, 669).

Aus der deutschen Offenlegungsschrift Nr. 2801316 sind bereits N-Phenyl-N'-(2-chlor-6-fluorbenzoyl)-harnstoffe mit insektizider Wirkung bekannt, deren Phenylrest mit einer Halogenalkoxygruppe in 3- oder 4-Stellung sowie gegebenenfalls einem Chloratom oder einer 3-$CF_3$-Gruppe substituiert ist. Aus der

europäischen Patentanmeldung Nr. 23.884 sind Insektizide ähnlicher Struktur bekannt, die einen N-4-Halogenalkoxyphenylrest mit einer fixierten $CF_3$-Gruppe in 3-Stellung aufweisen. Demgegenüber sind die neuartigen erfindungsgemässen 4-Halogenalkoxyphenylbenzoylharnstoffe im wesentlichen dadurch charakterisiert, dass sie am Phenylrest mit einer $CF_3$-Gruppe substituiert sind und ausserdem in 3- und/oder 5-Stellung einen Substituenten besitzen.

Es wurde überraschenderweise gefunden, dass die erfindungsgemässen Verbindungen der Formel I bei guter Pflanzenverträglichkeit und geringer Warmblütertoxizität ausgezeichnete Wirksamkeit als Schädlingsbekämpfungsmittel aufweisen. Sie eignen sich vor allem zur Bekämpfung von Pflanzen und Tiere befallenden Schädlingen.

Insbesondere eignen sich die Verbindungen der Formel I zur Bekämpfung von Insekten der Ordnungen Lepidoptera, Coleoptera, Homoptera, Heteroptera, Diptera, Thysanoptera, Orthoptera, Anoplura, Siphonaptera, Mallophaga, Thysanura, Isoptera, Psocoptera und Hymenoptera sowie von Vertretern der Ordnung Akarina.

Die gute pestizide Wirkung der erfindungsgemässen Verbindungen der Formel I entspricht einer Abtötungsrate (Mortalität) von mindestens 50-60% der erwähnten Schädlinge.

Neben ihrer sehr günstigen Wirkung gegenüber Fliegen, wie z.B. Musca domestica, und Mückenlarven eignen sich Verbindungen der Formel I vor allem zur Bekämpfung von pflanzenschädigenden Frassinsekten, in Zier- und Nutzpflanzungen, insbesondere in Baumwollkulturen (z.B. Spodoptera littoralis und Heliothis virescens) sowie in Gemüsekulturen (z.B. Leptinotarsa decemlineata, Pieris brassicae und Plutella xylostella). Hervorzuheben ist besonders die larvizide und ovizide Wirkung von Verbindungen der Formel I. Werden Verbindungen der Formel I von adulten Insekten mit dem Futter aufgenommen, so ist in vielen Fällen, insbesondere bei Coleopteren, wie z.B. Anthonomus grandis, eine verminderte Ei-Ablage und/oder reduzierte Schlupfrate festzustellen.

Die Verbindungen der Formel I eignen sich weiterhin zur Bekämpfung von Ektoparasiten, z.B. Lucilia sericata, sowie von Zecken an Haus- und Nutztieren, z.B. durch Tier-, Stall- und Weidebehandlung.

Die Wirkung der erfindungsgemässen Verbindungen bzw. der sie enthaltenden Mittel lässt sich durch Zusatz von anderen Insektiziden und/oder Akariziden wesentlich verbreitern und an gegebene Umstände anpassen. Als Zusätze kommen z.B. Vertreter der folgenden Wirkstoffklassen in Betracht; Organische Phosphorverbindungen, Nitrophenole und Derivate, Formamidine, Harnstoffe, Carbamate, Pyrethroide, chlorierte Kohlenwasserstoffe und Bacillus thuringiensis-Präparate.

Die Verbindungen der Formel I werden in unveränderter Form oder vorzugsweise zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren, wie Versprühen, Vernebeln, Verstäuben, Verstreuen oder Giessen, werden ebenso wie die Mittel den angestrebten Zielen und den gegebenen Verhältnissen entsprechend gewählt.

Die Formulierung, d.h. die den Wirkstoff der Formel I, bzw. Kombinationen dieser Wirkstoffe mit anderen Insektiziden oder Akariziden, und gegebenenfalls einen festen oder flüssigen Zusatzstoff enthaltenden Mittel, Zubereitungen oder Zusammensetzungen, werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester, wie Dibutyl- oder Dioctylphthalat, aliphatische Kohlenwasserstoffe, wie Cyclohexan, Paraffine, Alkohole und Glykole sowie deren Aether und Ester, wie Aethanol, Aethylenglykol, Aethylenglykolmonomethyl- oder -äthyläther, Ketone, wie Cyclohexanon, stark polare Lösungsmittel, wie N-Methyl-2-pyrrolidon, Dimethylsulfoxid oder Diemthylformamid, sowie gegebenenfalls epoxidierte Pflanzenöle, wie epoxidiertes Kokosnussöl oder Sojaöl, oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäuren oder hochdisperse saugfähige Polymerisate zugesetzt werden. Als gekörnte, adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüberhinaus kann eine Vielzahl von granulierten Materialien anorganischer oder organischer Natur, wie insbesondere Dolomit oder zerkleinerte Pflanzenrückstände, verwendet werden.

Als oberflächenaktive Verbindungen kommen je nach Art des zu formulierenden Wirkstoffes der Formel I oder der Kombinationen dieser Wirkstoffe mit andern Insektiziden oder Akariziden nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Tallöl gewonnen werden können. Ferner sind als Tenside auch die Fettsäuremethyl-taurin-salze sowie modifizierte und nicht modifizierte Phospholipide zu erwähnen.

Häufiger werden jedoch sogenannte synthetische Tenside verwendet, insbesondere Fettsulfonate,

Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen im allgemeinen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit etwa 8-22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes. Ferner kommen auch entsprechende Phosphate, wie z.B. Salze des Phosphorsäureesters eines p-Nonylphenol-(4-14)-Aethylenoxid-Adduktes, in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können. Weiterhin geeignete nichtionische Tenside sind die wasserlöslichen 20 bis 250 Aethylenglykoläthergruppen und 10 bis 100 Propylenglykoläthergruppen enthaltenden Polyäthylenoxid-Addukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiel nichtionischer Tenside seien Nonylphenolpolyäthoxyäthanole, Ricinusölpolyglykoläther, Polypropylen-Polyäthylenoxid-Addukte, Tributylphenoxypolyäthoxyäthanol, Polyäthylenglykol und Octylphenoxypolyäthoxyäthanol erwähnt. Ferner kommen auch Fettsäureester von Polyoxyäthylensorbitan, wie das Polyoxyäthylensorbitantrioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyl-di-(2-chloräthyl)äthylammoniumbromid.

Die in der Formulierungstechnik gebräuchlichen Tenside sind u.a. in folgenden Publikationen beschrieben: "Mc Cutcheon's Detergents and Emulsifiers Annual" MC Publishing Corp., Ridgewood, New Jersey, 1979; Dr. Helmut Stache "Tenside Taschenbuch", Carl Hanser Verlag München/Wien 1981.

Die pestiziden Zubereitungen enthalten in der Regel 0,1 bis 99 %, insbesondere 0,1 bis 95 %, Wirkstoff der Formel I oder Kombinationen dieser Wirkstoffe mit andern Insektiziden oder Akariziden, 1 bis 99,9 % eines festen oder flüssigen Zusatzstoffes und 0 bis 25 %, insbesondere 0,1 bis 20 %, eines Tensides. Während als Handelsware eher konzentrierte Mittel bevorzugt werden, verwendet der Endverbraucher in der Regel verdünnte Zubereitungen, die wesentlich geringere Wirkstoffkonzentrationen aufweisen.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Beispiel 1: a) Herstellung der Ausgangsverbindung 2-Chlor-4-(1,1,2,3,3,3-hexafluorpropyloxy)-5-(trifluormethyl)-anilin:

Es werden 29,6 g 2-Chlor-4-(1,1,2,3,3,3-hexafluorpropyloxy)-5-(trifluormethyl)-acetaminobenzol in einer Mischung aus 100 ml konzentrierter Salzsäure und 400 ml Aethanol während 17 Stunden am Rückfluss gekocht. Anschliessend wird die Hauptmenge des Lösungsmittels abdestilliert, mit 10 %iger Natronlauge alkalisch gestellt und mit Aether extrahiert. Nach Trocknen über Natriumsulfat und Verdampfen des Lösungsmittels im Vakuum wird der Rückstand fraktioniert destilliert. Man erhält die Titelverbindung als farbloses Oel mit einem Siedepunkt von 81-83°C / $4 \cdot 10^{-2}$Torr.

b) Herstellung der Ausgangsverbindungen 2-Chlor-4-(1,1,2,3,3,3-hexafluorpropyloxy)-5-(trifluormethyl)- und 2-Chlor-3-(trifluormethyl)-4-(1,1,2,3,3,3-hexafluorpropyloxy)-anilin:

15 g 3-Trifluormethyl-4-(1,1,2,3,3,3-hexafluorpropyloxy)-anilin werden in 50 ml Acetonitril vorgelegt und zu diesem Gemisch werden bei 60°C in einer Portion 6,7 g N-Chlorsuccinimid zugesetzt. Nach dem Abklingen der exothermen Reaktion wird der Ansatz noch 2 Stunden am Rückfluss erhitzt, anschliessend das Lösungsmittel in Vakuum abdestilliert und der Rückstand in Methylenchlorid aufgenommen. Die erhaltene Lösung wird zweimal mit je 50 ml 10%iger Natronlauge gewaschen, mit Kochsalzlösung nachgewaschen und über Natriumsulfat getrocknet. Nach Entfernen des Lösungsmittels durch Abdampfen wird der flüssige Rückstand an Kieselgel chromatographiert (Laufmittel: Hexan/Essigester = 4:1). Man erhält so das 2-Chlor-4-(1,1,2,3,3,3-hexafluorpropyloxy)-5-trifluormethylanilin (Siedepunkt 81-83°C/$4 \cdot 10^{2}$Torr) sowie etwa die doppelte Menge an 2-Chlor-3-trifluormethyl-4-(1,1,2,3,3,3-hexafluorpropyloxy)-anilin ($n_D^{26}$ = 1,4340) als farbloses Oel.

Die entsprechend der vorstehend beschriebenen Arbeitsweisen hergestellten Verbindungen der Formel II sind in folgender Tabelle zusammengefasst:

| $R_3$ | $R_4$ | $R_5$ | R | physik. Daten |
|---|---|---|---|---|
| $-CF_3$ | Cl | H | $-CF_2-CHF-CF_3$ | Sdp.120°/$6\cdot10^{-2}$Torr |
| Cl | $-CF_3$ | Cl | $-CF_2-CHF-CF_3$ | Sdp.130°/$4\cdot10^{-2}$Torr |
| Br | H | $-CF_3$ | $-CF_2-CHF-CF_3$ | $n_D^{21}$ =1,4450 |
| $-CF_3$ | H | Cl | $-CF_2-CHFCl$ | Sdp.120°C/$5\cdot10^{-2}$Torr |
| $-CF_3$ | Cl | Cl | $-CF_2-CHF-CF_3$ | Sdp.130°C/$6\cdot10^{-2}$Torr |
| $-CF_3$ | H | Cl | $-CF_2-CHF-CF_3$ | Sdp.64-66°C/$3\cdot10^{-2}$Torr |
| $-CF_3$ | Cl | Cl | $-CF_2-CHFCl$ | $n_D^{20}$=1,5017 |
| Cl | H | $-CF_3$ | $-CF_2-CHF-CF_3$ | Sdp.81-83°C/$4\cdot10^{-2}$Torr<br>$n_D^{24}$ =1,4278 |
| Cl | $-CF_3$ | H | $-CF_2-CHF-CF_3$ | $n_D^{26}$ =1,4340 |
| Cl | H | $-CF_3$ | $-CF_2-CHFCl$ | Sdp. 88-90°C/$3\cdot10^{-2}$Torr<br>$n_D^{25}$ =1,4709 |
| Cl | Cl | $-CF_3$ | $-CF_2-CHF-CF_3$ | $n_D^{23}$ =1,4535 |

Herstellbar sind ferner gemäss der obigen Arbeitsweisen die folgenden Verbindungen der Formel II, worin die Reste $R_3$, $R_4$, $R_5$ und R die in der nachstehenden Tabelle angegebenen Bedeutungen haben:

| $R_3$ | $R_4$ | $R_5$ | R |
|---|---|---|---|
| Cl | Cl | $-CF_3$ | $-CF_2-CHF-CF_3$ |
| Br | H | $-CF_3$ | $-CF_2-CHF-CF_3$ |
| $-CF_3$ | Cl | Cl | $-CF_2-CHF_2$ |
| Cl | $-CF_3$ | Cl | $-CF_2-CHCl_2$ |
| Cl | $-CF_3$ | H | $-CF_2-CHFBr$ |
| Br | Cl | $-CF_3$ | $-CF_2-CHF_2$ |
| $-CF_3$ | H | Br | $-CF_2-CHF-CF_3$ |
| $-CF_3$ | Cl | Cl | $-CF_2-CHF-CF_3$ |
| $-CF_3$ | Cl | Br | $-CF_2-CHF-CF_3$ |
| Br | $-CF_3$ | H | $-CF_2-CHF-CF_3$ |
| Cl | $-CF_3$ | Cl | $-CF_2-CHF-CF_3$ |
| $-CF_3$ | H | $-CF_3$ | $-CF_2-CHF-CF_3$ |

Beispiel 2: Herstellung von N-[2-Chlor-5-trifluormethyl-4-(hexafluorpropyloxy)-phenyl]-N'-(2,6-difluorbenzoyl)-harnstoff:

Es werden 3,0 g des nach Beispiel 1a erhaltenen 2-Chlor-4-(1,1,2,3,3,3-hexafluorpropyloxy)-5-(trifluormethyl)-anilins in 10 ml trockenem Diäthyläther vorgelegt. Unter Rühren werden bei Raumtemperature 1,5 g 2,6-Difluorbenzoylisocyanat zugetropft. Nach 2 Stunden wird der gebildete kristalline Niederschlag abfiltriert und mit wenig Aether ausgewaschen. Man erhält so die Titelverbindung der Formel

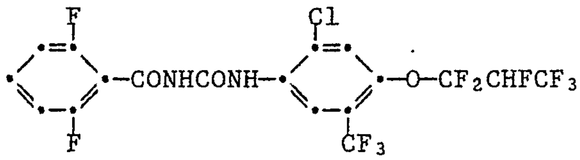

als weisse Kristalle mit einem Schmelzpunkt von 158-159°C (Verbindung Nr. 1).

Entsprechend der vorstehend beschriebenen Arbeitsweisen werden auch die folgenden Verbindungen der Formel I hergestellt:

| Verbindung Nr. | $R_1$ | $R_2$ | $R_3$ | $R_4$ | $R_5$ | R | Smp. [°C] |
|---|---|---|---|---|---|---|---|
| 2 | H | F | Cl | H | $-CF_3$ | $-CF_2-CHF-CF_3$ | 132-133,5 |
| 3 | F | Cl | Cl | H | $-CF_3$ | $-CF_2-CHF-CF_3$ | 185-187 |
| 4 | H | Cl | Cl | H | $-CF_3$ | $-CF_2-CHF-CF_3$ | 166-168 |
| 5 | F | F | Cl | $-CF_3$ | H | $-CF_2-CHF-CF_3$ | 150-152 |
| 6 | H | Cl | Cl | $-CF_3$ | H | $-CF_2-CHF-CF_3$ | 171,5-172,5 |
| 7 | F | Cl | Cl | $-CF_3$ | H | $-CF_2-CHF-CF_3$ | 155-156 |
| 8 | F | F | Cl | H | $-CF_3$ | $-CF_2-CHFCl$ | 170-171,5 |
| 9 | F | Cl | Cl | H | $-CF_3$ | $-CF_2-CHFCl$ | 198-199 |
| 10 | F | H | Cl | H | $-CF_3$ | $-CF_2-CHFCl$ | 142-143,5 |
| 11 | Cl | H | Cl | H | $-CF_3$ | $-CF_2-CHFCl$ | 167-168,5 |
| 12 | F | H | $-CF_3$ | H | Cl | $-CF_2-CHF-CF_3$ | 120-121,5 |
| 13 | F | H | $-CF_3$ | H | Cl | $-CF_2-CHFCl$ | 136-137 |
| 14 | F | F | $-CF_3$ | H | Cl | $-CF_2-CHF-CF_3$ | 148-150 |
| 15 | F | F | $-CF_3$ | H | Cl | $-CF_2-CHFCl$ | 150,5-152 |
| 16 | F | F | $-CF_3$ | Cl | Cl | $-CF_2-CHF-CF_3$ | 168-169 |
| 17 | F | F | $-CF_3$ | Cl | H | $-CF_2-CHF-CF_3$ | 148-150 |
| 18 | Cl | H | $-CF_3$ | Cl | Cl | $-CF_2-CHF-CF_3$ | 163-164,5 |
| 19 | Cl | H | $-CF_3$ | H | Cl | $-CF_2-CHFCl$ | 142-143,5 |
| 20 | Cl | H | $-CF_3$ | H | Cl | $-CF_2-CHF-CF_3$ | 145-146,5 |
| 21 | F | F | $-CF_3$ | Cl | Cl | $-CF_2-CHFCl$ | 158-159 |
| 22 | Cl | H | $-CF_3$ | Cl | Cl | $-CF_2-CHFCl$ | 153-154 |
| 23 | F | F | Cl | $-CF_3$ | Cl | $-CF_2-CHF-CF_3$ | 156,5-158 |
| 24 | F | H | Cl | $-CF_3$ | H | $-CF_2-CHF-CF_3$ | 161-162 |
| 25 | F | F | Br | H | $-CF_3$ | $-CF_2-CHF-CF_3$ | 157-158 |
| 26 | Cl | H | Br | H | $-CF_3$ | $-CF_2-CHF-CF_3$ | 173,5-174 |
| 27 | F | F | Cl | Cl | $-CF_3$ | $-CF_2-CHF-CF_3$ | 149,5-151 |
| 28 | F | $-OCH_3$ | Br | H | $-CF_3$ | $-CF_2-CHF-CF_3$ | 187-188,5 |
| 29 | F | H | Br | H | $-CF_3$ | $-CF_2-CHF-CF_3$ | 136-137 |

Die nachstehenden Verbindungen der Formel I sind ebenfalls entsprechend den oben angegebenen Arbeitsweisen erhältlich:

| $R_1$ | $R_2$ | $R_3$ | $R_4$ | $R_5$ | R |
|---|---|---|---|---|---|
| F | $-OCH_3$ | $-CF_3$ | Cl | H | $-CF_2-CHF-CF_3$ |
| F | $-OCH_3$ | Cl | H | $-CF_3$ | $-CF_2-CHF-CF_3$ |
| F | $-OCH_3$ | Cl | $-CF_3$ | H | $-CF_2-CHF-CF_3$ |
| $-OCH_3$ | $-OCH_3$ | Br | H | $-CF_3$ | $-CF_2-CHF-CF_3$ |
| $-OCH_3$ | $-OCH_3$ | Cl | H | $-CF_3$ | $-CF_2-CHF-CF_3$ |
| F | F | $-CF_3$ | Cl | Cl | $-CF_2-CHF_2$ |
| F | F | $-CF_3$ | H | Cl | $-CF_2-CHFCl$ |
| F | F | Cl | $-CF_3$ | Cl | $-CF_2-CHCl_2$ |
| F | F | Cl | $-CF_3$ | H | $-CF_2-CHFBr$ |
| F | F | Br | Cl | $-CF_3$ | $-CF_2-CHF_2$ |
| H | Cl | Cl | H | $-CF_3$ | $-CF_2-CHFCl$ |
| Cl | F | Cl | Cl | $-CF_3$ | $-CF_2-CHF-CF_3$ |
| Cl | H | Cl | Cl | $-CF_3$ | $-CF_2-CHF-CF_3$ |
| Cl | F | $-CF_3$ | Cl | Cl | $-CF_2-CHF-CF_3$ |
| Cl | F | $-CF_3$ | Cl | Cl | $-CF_2-CHFCl$ |
| Cl | H | Cl | $-CF_3$ | Cl | $-CF_2-CHF-CF_3$ |
| Cl | F | Cl | $-CF_3$ | Cl | $-CF_2-CHF-CF_3$ |
| F | F | Cl | $-CF_3$ | Cl | $-CF_2-CHFCl$ |
| F | Cl | $-CF_3$ | Cl | H | $-CF_2-CHF-CF_3$ |
| Cl | H | $-CF_3$ | Cl | H | $-CF_2-CHF-CF_3$ |
| F | Cl | $-CF_3$ | H | Cl | $-CF_2-CHF-CF_3$ |
| F | Cl | $-CF_3$ | H | Cl | $-CF_2-CHFCl$ |
| F | F | $-CF_3$ | H | Cl | $-CF_2-CHF_2$ |
| F | F | $-CF_3$ | H | Cl | $-CF_2-CHFBr$ |
| Cl | H | $-CF_3$ | H | Cl | $-CF_2-CHFBr$ |
| Cl | H | $-CF_3$ | H | Cl | $-CF_2-CHF_2$ |
| F | F | $-CF_3$ | H | Br | $-CF_2-CHF-CF_3$ |
| F | Cl | $-CF_3$ | H | Br | $-CF_2-CHF-CF_3$ |
| Cl | H | $-CF_3$ | H | Br | $-CF_2-CHF-CF_3$ |
| F | F | F | Cl | $CF_3$ | $-CF_2-CHF-CF_3$ |

| $R_1$ | $R_2$ | $R_3$ | $R_4$ | $R_5$ | R |
|---|---|---|---|---|---|
| F | F | $-CF_3$ | H | Br | $-CF_2-CHFCl$ |
| F | F | $-CF_3$ | H | Br | $-CF_2-CHF_2$ |
| F | F | $-CF_3$ | H | Br | $-CF_2-CHFBr$ |
| F | F | F | $-CF_3$ | Cl | $-CF_2-CHF-CF_3$ |
| F | F | F | H | $-CF_3$ | $-CF_2-CHF-CF_3$ |
| F | F | F | $-CF_3$ | H | $-CF_2-CHF-CF_3$ |
| F | Cl | F | H | $-CF_3$ | $-CF_2-CHF-CF_3$ |
| Cl | H | F | H | $-CF_3$ | $-CF_2-CHF-CF_3$ |
| H | F | Cl | H | $-CF_3$ | $-CF_2-CHFCl$ |
| F | F | $-CF_3$ | Cl | Br | $-CF_2-CHF-CF_3$ |
| H | Cl | Br | $-CF_3$ | H | $-CF_2-CHF-CF_3$ |

Beispiel 3: Formulierungen für Wirkstoffe der Formel I gemäss den Beispiel 2 resp. Kombinationen dieser Wirkstoffe mit andern Insektiziden oder Akariziden (% = Gewichtsprozent):

1. Spritzpulver

| | a) | b) | c) |
|---|---|---|---|
| Wirkstoff oder Wirkstoffkombination | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykoläther (7-8 Mol AeO) | - | 2 % | - |
| Hochdisperse Kieselsäure | 5 % | 10 % | 10 % |
| Kaolin | 62 % | 27 % | - |

Der Wirkstoff oder die Wirkstoffkombination wird mit den Zusatzstoffen vermischt und in einer geeigneten Mühle gut vermahlen.

Man erhält Spritzpulver, die sich mit Wasser zu Suspensionen jeder gewünschten Konzentration verdünnen lassen.

2. Emulsions-Konzentrat

Wirkstoff oder Wirkstoffkombination 10 %
Octylphenolpolyäthylenglykoläther (4-5 Mol AeO) 3 %
Ca-Dodecylbenzolsulfonat 3 %
Ricinusölpolyglykoläther (36 Mol AeO) 4 %
Cyclohexanon 30 %
Xylolgemisch 50 %

Aus diesem Konzentrat können durch Verdünnen mit Wasser Emulsionen jeder gewünschten Konzentration hergestellt werden.

3. Stäubemittel

| | a) | b) |
|---|---|---|
| Wirkstoff oder Wirkstoffkombination | 5 % | 8 % |
| Talkum | 95 % | - |
| Kaolin | - | 92 % |

Man erhält anwendungsfertige Stäubemittel, indem der Wirkstoff mit dem Träger vermischt und auf einer geeigneten Mühle vermahlen wird.

4. Extruder-Granulat

Wirkstoff oder Wirkstoffkombination 10 %
Na-Ligninsulfonat 2 %
Carboxymethylcellulose 1 %
Kaolin 87 %

Der Wirkstoff wird mit den Zusatzstoffen vermischt, vermahlen und mit Wasser angefeuchtet. Dieses Gemisch wird extrudiert, granuliert und anschliessend im Luftstrom getrocknet.

5. Umhüllungs-Granulat

Wirkstoff oder Wirkstoffkombination 3 %
Polyäthylenglykol (MG 200) 3 %
Kaolin 94 %

Der fein gemahlene Wirkstoff oder die Wirkstoffkombination wird in einem Mischer auf das mit Polyäthylenglykol angefeuchtete Kaolin gleichmässig aufgetragen. Auf diese Weise erhält man staubfreie Umhüllungs-Granulate.

6. Suspensions-Konzentrat

Wirkstoff oder Wirkstoffkombination 40 %
Aethylenglykol 10 %
Nonylphenolpolyäthylenglykoläther (15 Mol AeO) 6 %
Na-Ligninsulfonat 10 %
Carboxymethylcellulose 1 %
37%ige wässrige Formaldehyd-Lösung 0,2 %
Silikonöl in Form einer 75%igen wässrigen Emulsion 0,8 %
Wasser 32 %

Der fein gemahlene Wirkstoff oder die Wirkstoffkombination wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Beispiel 4: Wirkung gegen Musca domestica

Je 50 g frisch zubereitetes Nährsubstrat für Maden werden in Becher eingewogen. Von einer 1 Gew.-%igen acetonischen Lösung des betreffenden Wirkstoffes wird eine bestimmte Menge auf das in den Bechern befindliche Nährsubstrat pipettiert, so dass sich eine Wirkstoffkonzentration von 400 ppm ergibt. Nach dem Durchmischen des Substrates lässt man das Aceton mindestens 20 Stunden lang verdampfen.

Dann werden pro Wirkstoff je 25 eintägige Maden von Musca domestica in die das so behandelte Nährsubstrat enthaltenden Becher gegeben. Nachdem sich die Maden verpuppt haben, werden die gebildeten Puppen durch Ausschwemmen mit Wasser von dem Substrat abgetrennt und in mit Siebdeckeln verschlossenen Gefässen deponiert.

Die pro Ansatz ausgeschwemmten Puppen werden gezählt (toxischer Einfluss des Wirkstoffes auf die Madenentwicklung). Dann wird nach 10 Tagen die Anzahl der aus den Puppen geschlüpften Fliegen bestimmt.

Verbindungen der Formel I gemäss den Beispiel 2 zeigen gute Wirkung im obigen Test.

Beispiel 5: Wirkung gegen Lucilia sericata

Zu 9 ml eines Zuchtmediums wird bei 50°C 1 ml einer 0,5 % Aktivsubstanz enthaltenden wässrigen Zubereitung gegeben. Nun werden ca. 30 frisch geschlüpfte Lucilia sericata-Larven zum Zuchtmedium gegeben. Nach 48 und 96 Stunden wird die insektizide Wirkung durch Ermittlung der Abtötungsrate festgestellt.

Verbindungen der Formel I gemäss Beispiel 2 zeigen in diesem Test gute Wirkung gegen Lucilia sericata.

Beispiel 6: Wirkung gegen Aëdes aegypti

Auf die Oberfläche von 150 ml Wasser, das sich in einem Behälter befindet, wird so viel einer 0,1%igen acetonischen Lösung des Wirkstoffes pipettiert, dass eine Konzentration von 400 ppm erhalten wird. Nach

Verdunsten des Acetons wird der Behälter mit 30 bis 40 2-tägigen Aëdes-Larven beschickt. Nach 2 und 7 Tagen wird die Mortalität geprüft.

Verbindungen der Formel I gemäss Beispiel 2 zeigen gute Wirkung im obigen Test.

Beispiel 7: Insektizide Frassgift-Wirkung

Baumwollpflanzen (ca. 20 cm hoch) werden mit wässrigen Wirkstoffemulsionen (erhalten aus einem 10%igen emulgierbaren Konzentrat) besprüht, wobei die Wirkstoffemulsionen 0,75 bis 50 ppm der jeweils zu prüfenden Verbindung enthalten.

Nach dem Antrocknen des Belages werden die Baumwollpflanzen mit Spodoptera littoralis- und Heliothis virescens-Larven im dritten larvalen Stadium besetzt. Der Versuch wird bei 24°C und 60 % relativer Luftfeuchtigkeit durchgeführt. In Abständen von jeweils 24 Stunden werden Mortalität sowie Entwicklungs- und Häutungsstörungen der angesetzten Larven bestimmt.

Gegen Spodoptera und Heliothis zeigt eine 80-100%ige Wirkung in diesem Test die Verbindung Nr. 14 bei 0,75 ppm bzw. 3 ppm.

Beispiel 8: Wirkung auf Spodoptera littoralis und Heliothis virescens (Larven und Eier)

Es werden drei in Töpfen gezogene Baumwollpflanzen von ca. 15-20 cm Höhe mit einer sprühfähigen flüssigen Zubereitung des zu prüfenden Wirkstoffes in einer Konzentration von 400 ppm behandelt. Nach Antrocknen des Sprühbelages werden die eingetopften Pflanzen in ein Blechgefäss von etwa 20 Litern Inhalt gestellt, das mit einer Glasplatte abgedeckt wird. Die Feuchtigkeit im Innern des abgedeckten Gefässes wird so reguliert, dass sich kein Kondenswasser bilden kann. Direktes, auf die Pflanzen fallendes Licht wird vermieden. Dann werden die drei Pflanzen infestiert, und zwar insgesamt mit:

a) 50 Larven von Spodoptera littoralis oder Heliothis virescens des ersten larvalen Stadiums;

b) 20 Larven von Spodoptera littoralis oder Heliothis virescens des dritten larvalen Stadiums;

c) zwei Eispiegeln von Spodoptera littoralis oder Heliothis virescens (dazu werden je 2 Blätter einer der Baumwoll-Pflanzen in einem beidseitig mit Gaze verschlossenen Plexiglaszylinder eingeschlossen); zwei Eispiegel von Spodoptera oder ein Teil eines Baumwollblates mit darauf abgelegten Eiern von Heliothis werden zu den eingeschlossenen Blättern gegeben.

Nach 4 und 5 Tagen erfolgt die Auswertung gegenüber unbehandelten Kontrollen unter Berücksichtigung folgender Kriterien:

a) Anzahl der noch lebenden Larven,

b) larvale Entwicklungs- und Häutungsstörungen,

c) Frassschaden (Schabfrass und Lochfrass),

d) Schlupfrate (Anzahl der aus den Eiern geschlüpften Larven).

Verbindungen der Formel I gemäss Beispiel 2 zeigen gute Gesamt-Wirksamkeit in obigem Test.

Beispiel 9: Ovizide Wirkung auf Spodoptera littoralis

Auf Filterpapier abgelegte Eier von Spodoptera littoralis werden aus dem Papier ausgeschnitten und in eine 0,05 Gew.%ige Lösung des Wirkstoffes in einem Aceton-Wasser-Gemisch (1:1) getaucht. Die so behandelten Eiablagen werden dann aus diesem Gemisch herausgenommen und bei 28°C und 60 % relativer Feuchtigkeit in Kunststoffschalen deponiert.

Nach 5 Tagen wird die Schlupfrate, d.h. die Anzahl Larven, die sich aus den behandelten Eiern entwickelt haben, bestimmt.

Verbindungen der Formel I gemäss den Beispiel 2 zeigen gute Wirkung im obigen Test.

Beispiel 10: Ovizide Wirkung auf Epilachna varivestis

Es werden 20 Gew.% Wirkstoff, 70 Gew.% Xylol und 10 Gew.% einer Mischung aus einem Reaktionsprodukt eines Alkylphenols mit Aethylenoxid und Calcium-dodecylbenzolsulfonat miteinander vermischt. Aus diesem Konzentrat werden wässrige Emulsionen enthaltend 800 ppm Wirkstoff hergestellt.

Jeweils ca. 100 auf Blätter von Phaseolus vulgaris frisch abgelegte Eier von Epilachna varivestis (mexikanischer Bohnenkäfer) werden mit den oben beschriebenen wässrigen Emulsionen (Konzentration 800 ppm Wirkstoff) angefeuchtet und leicht getrocknet.

In einem gelüfteten Gefäss werden die behandelten Gelege so lange gehalten, bis die gleichzeitig angesetzten unbehandelten Kontrollen geschlüpft sind. Unter einem Binocular erfolgt Auswertung hinsichtlich der erzielten prozentualen Abtötung.

Verbindungen der Formel I gemäss Beispiel 2 zeigen gute Wirkung in obigem Test.

Beispiel 11: Ovizide Wirkung auf Heliothis virescens und Leptinotarsa decemlineata

Entsprechende Mengenanteile einer benetzbaren pulverförmigen Formulierung, enthaltend 25 Gew.% des zu prüfenden Wirkstoffes, werden mit jeweils so viel Wasser vermischt, dass sich eine wässrige Emulsion mit einer Wirkstoffkonzentration von 800 ppm ergibt.

In die wirkstoffhaltigen Emulsionen werden eintägige Eigelege von Heliothis auf Cellophan® bzw. Eigelege von Leptinotarsa auf Kartoffelblättern während drei Minuten eingetaucht und dann auf Rundfiltern abgenutscht. Die so behandelten Gelege werden in Petrischalen ausgelegt und in der Dunkelheit aufbewahrt. Nach 6 bis 8 Tagen wird die Schlupfrate im Vergleich zu unbehandelten Kontrollen festgelegt.

Verbindungen der Formel I gemäss Beispiel 2 zeigen gute Wirkung in obigem Test.

Beispiel 12: Ovizide Wirkung auf Laspeyresia pomonella (Eier)

Abgelegte Eier von Laspeyresia pomonella, die nicht älter als 24 Stunden sind, werden auf Filterpapier für 1 Minute in eine acetonisch-wässrige Lösung enthaltend 400 ppm des zu prüfenden Wirkstoffes eingetaucht. Nach dem Antrocknen der Lösung werden die Eier in Petrischalen ausgelegt und bei einer Temperatur von 28°C belassen. Nach 6 Tagen wird der prozentuale Schlupf aus den behandelten Eiern bewertet.

Verbindungen der Formel I gemäss den Beispiel 2 zeigen gute Wirkung in obigem Test.

Beispiel 13: Wirkung gegen Anthonomus grandis (Adulte)

Zwei eingetopfte Baumwollpflanzen im 6-Blattstadium werden mit wässrigen benetzungsfähigen Emulsions-Zubereitungen, enthaltend 400 ppm des zu prüfenden Wirkstoffes besprüht. Nach dem Antrocknen des Spritzbelages (etwa 1,5 Stunden) wird jede Pflanze mit 10 adulten Käfern (Anthonomus grandis) besiedelt. Plastikzylinder, deren obere Oeffnungen mit Gaze abgedeckt sind, werden dann über die behandelten, mit den Testtieren besiedelten Pflanzen gestülpt, um ein Abwandern der Käfer zu verhindern. Die behandelten Pflanzen werden bei 25°C und etwa 60 % relativer Luftfeuchtigkeit gehalten. Die Auswertung erfolgt nach 2, 3, 4 und 5 Tagen unter Zugrundelegung der prozentualen Mortalität der eingesetzten Testtiere (% Rückenlage) sowie der Antifeedant-Wirkung gegenüber unbehandelten Kontrollansätzen.

Verbindungen der Formel I gemäss Beispiel 2 zeigen gute Wirkung im obigen Test.

Beispiel 14: Wirkung gegen pflanzenschädigende Akariden: Tetranychus urticae (OP-sensible) und Tetranychus cinnabarinus (OP-tolerant).

Die Primärblätter von Phaseolus vulgaris-Pflanzen werden 16 Stunden vor dem Versuch auf akarizide Wirkung mit einem infestierten Blattstück aus einer Massenzucht von Tetranychus urticae (OP-sens.) oder Tetranchus cinnabarinus (OP-tol.) belegt. (Die Toleranz bezieht sich auf die Verträglichkeit gegenüber Diazinon).

Die so behandelten infestierten Pflanzen werden mit einer Versuchslösung enthaltend 400 ppm der zu prüfenden Verbindung bis zur Tropfnässe besprüht.

Nach 24 Stunden und wiederum nach 7 Tagen werden Imagines und Larven (alle beweglichen Stadien) unter dem Binokular auf lebende und tote Individuen ausgewertet.

Man verwendet pro Konzentration und pro Testspezies eine Pflanze. Während des Versuchsverlaufs stehen die Pflanzen in Gewächshauskabinen bei 25°C.

Verbindungen der Formel I gemäss Beispiel 2 zeigen in diesem Versuch gute Wirkung gegen Tetranychus urticae und Tetranychus cinnabarinus.

Beispiel 15: Reproduktions-Beeinflussung von Anthonomus grandis:

Adulte Anthonomus grandis, die nach dem Schlupf nicht älter als 24 Stunden waren, werden in Gruppen zu jeweils 25 Käfern in Käfige mit Gitterwänden überführt. Die mit den Käfern besetzen Käfige werden sodann während 5 bis 10 Sekunden in eine acetonische Lösung, enthaltend 800 ppm des zu prüfenden Wirkstoffes, eingetaucht. Nachdem die Käfer wieder trocken sind, werden sie zur Kopulation und Eiablage in abgedeckte und Futter enthaltende Schalen eingesetzt. Abgelegte Eier werden zwei- bis dreimal wöchentlich mit fliessendem Wasser ausgeschwemmt, gezählt, durch zwei- bis dreistündiges Einlegen in ein wässriges Desinfektionsmittel desinfiziert und dann in Schalen, die eine geeignete Larvaldiät enthalten, deponiert. Nach 7 Tagen wird untersucht, ob sich aus den deponierten Eiern Larven entwickelt haben.

Zur Ermittlung der Dauer des die Reproduktion beeinflussenden Effektes der zu prüfenden Wirkstoffe wird die Eiablage der Käfer während eines Zeitraums von etwa 4 Wochen überprüft. Die Bonitierung erfolgt anhand der Verminderung der Anzahl abgelegter Eier und der daraus geschlüpften Larven im Vergleich zu unbehandelten Kontrollen.

Verbindungen der Formel I gemäss Beispiel 2 zeigen eine gute reproduktionsreduzierende Wirkung im obigen Test.

Beispiel 16: Insektizide Frassgift-Wirkung gegen Plutella xylostella:

Eingetopfte Chinakohlpflanzen (Topfgrösse 10 cm Durchmesser) im 4-Blatt-Stadium werden mit wässrigen Wirkstoffemulsionen besprüht, die den Wirkstoff in einer Konzentration von 0,75 ppm enthalten und auf den Pflanzen antrocknen.

Nach zwei Tagen werden die behandelten Chinakohlpflanzen mit je 10 Plutella xylostella-Larven im zweiten larvalen Stadium besiedelt. Der Versuch wird bei 24°C und 60 % relativer Luftfeuchtigkeit im Dämmerlicht durchgeführt. Die Bonitur erfolgt nach 2 und 5 Tagen; es wird die %-Mortalität der Larven bestimmt.

Eine Wirkung von 80-100 % (Mortalität) zeigt die Verbindung der Nr. 14 gemäss Beispiel 2 in diesem Test.

**Patentansprüche**

1. Verbindung der Formel I

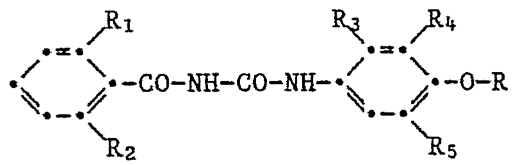

(I),

worin
R $C_1$-$C_4$-Halogenalkyl mit 1 bis 9 Halogenatomen;
$R_1$ Wasserstoff, Halogen, Methyl, Aethyl, Methoxy oder Aethoxy:
$R_2$ Halogen oder Methoxy;
$R_3$ Halogen oder Trifluormethyl; und
$R_4$ und $R_5$ unabhängig voneinander Wasserstoff, Halogen oder Trifluormethyl bedeuten, mit der Massgabe, dass mindestens einer der Reste $R_3$, $R_4$ und $R_5$ die Bedeutung Trifluormethyl besitzt und dass $R_4$ und $R_5$ nicht gleichzeitig Wasserstoff bedeuten.

2. Verbindung der Formel I gemäss Anspruch 1, dadurch gekennzeichnet, dass R mit 1 bis 7 Halogenatomen substituiertes $C_1$-$C_3$-Alkyl bedeutet.

3. Verbindung der Formel I gemäss Anspruch 1, dadurch gekennzeichnet, dass R mit 1 bis 9 Halogenatomen substituiertes $C_2$-$C_4$-Alkyl bedeutet.

4. Verbindung der Formel I gemäss einem der Ansprüche 1 bis 3, dadurch gekennzeichnet, dass $R_3$ Chlor oder Trifluormethyl; $R_4$ und $R_5$ unabhängig voneinander Wasserstoff, Chlor oder Trifluormethyl bedeuten.

5. Verbindung der Formel I gemäss einem der Ansprüche 1 bis 4, dadurch gekennzeichnet, dass $R_1$ Wasserstoff, Fluor oder Chlor und $R_2$ Fluor oder Chlor bedeuten.

6. Verbindung der Formel I gemäss Anspruch 5, dadurch gekennzeichnet, dass $R_1$ und $R_2$ unabhängig voneinander Fluor oder Chlor bedeuten.

7. Verbindung der Formel I gemäss Anspruch 6, dadurch gekennzeichnet, dass $R_1$ und $R_2$ gleichzeitig Fluor bedeuten.

8. Verbindung der Formel I gemäss einem der Ansprüche 1 bis 7 dadurch gekennzeichnet, dass R einen der Reste $-CHF_2$, $-CF_3$, $-CF_2CHF_2$, $-CH_2$-$CF_3$, $-CF_2CHFCl$, $-CF_2CHCl_2$ $-CF_2CCl_3$, $-CF_2CHBr_2$, $-CF_2CF_3$, $-CF_2CHFBr$, $-CF_2CHBr_2$, $-CH_2CHBrCH_2Br$ oder $-CF_2CHFCF_3$ bedeutet.

9. Verbindung der Formel I gemäss Anspruch 8, dadurch gekennzeichnet, dass R $-CF_2CHF_2$, $-CF_2CHFCl$, $CF_2CHFBr$ oder $CF_2CHFCF_3$ bedeutet.

10. Verbindung der Formel I gemäss Anspruch 9, dadurch gekennzeichnet, dass $R_3$ den Rest $-CF_2CHFCF_3$ bedeutet.

11. Verbindung gemäss Anspruch 10 der Formel

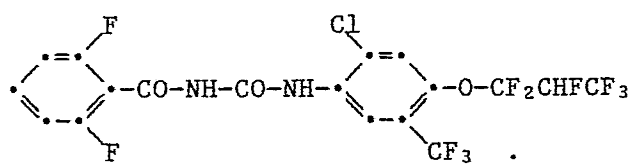

.

12. Verbindung gemäss Anspruch 10 der Formel

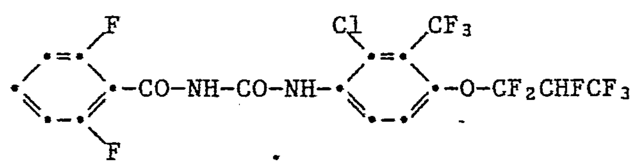

.

13. Verbindung gemäss Anspruch 9 der Formel

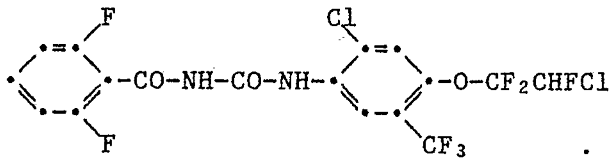

14. Verbindung gemäss Anspruch 10 der Formel

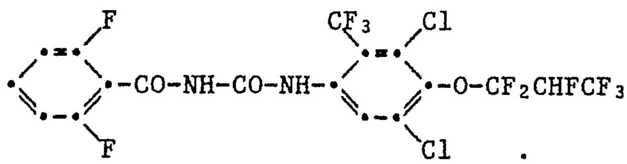

15. Verbindung gemäss Anspruch 10 der Formel

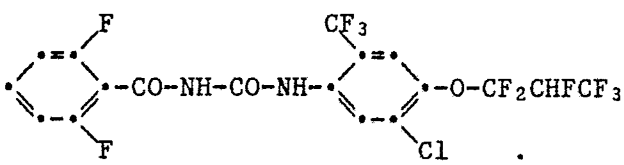

16. Verbindung gemäss Anspruch 10 der Formel

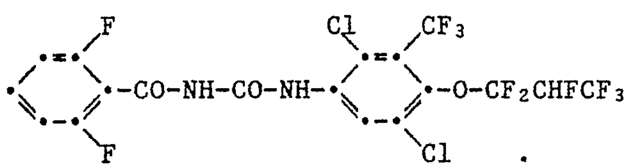

17. Verbindung gemäss Anspruch 10 der Formel

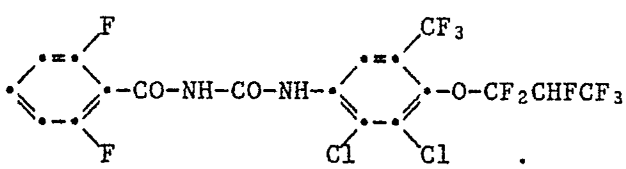

18. Verbindung gemäss Anspruch 10 der Formel

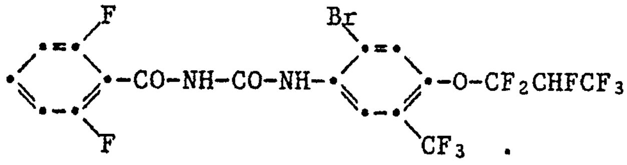

19. Verfahren zur Herstellung einer Verbindung gemäss einem der Ansprüche 1 bis 18, dadurch gekennzeichnet, dass man
a) eine Verbindung der Formel II

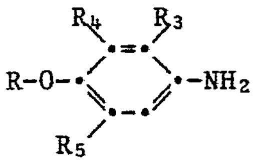

(II)

mit einer Verbindung der Formel III

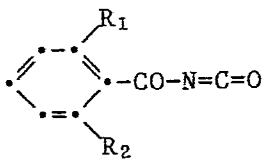

(III)

oder

b) eine Verbindung der Formel IV

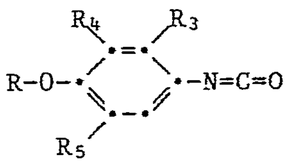

(IV)

mit einer Verbindung der Formel V

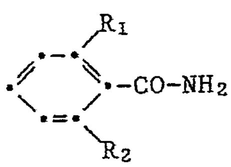

(V),

oder

c) eine Verbindung der Formel II mit einer Verbindung der Formel VI

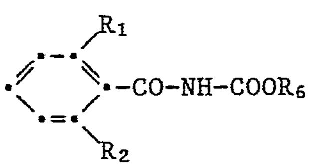

(VI)

umsetzt, wobei in den Formeln II bis VI die Reste R, $R_1$, $R_2$, $R_3$, $R_4$ und $R_5$ die in den Ansprüchen 1 bis 10 angegebenen Bedeutungen haben und $R_6$ für einen $C_1$-$C_8$-Alkylrest, der gegebenenfalls mit Halogen substituiert ist, steht.

20. Verbindung der Formel II

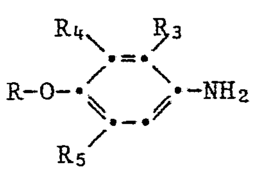

(II) ,

worin die Reste R, $R_3$, $R_4$ und $R_5$ die unter den Ansprüchen 1 bis 4 und 8 bis 10 angegebenen Bedeutungen haben.

21. Verbindung der Formel IV

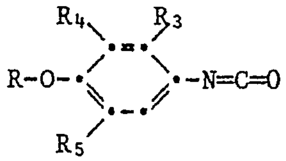

(IV) ,

worin die Reste R, $R_3$, $R_4$ und $R_5$ die unter den Ansprüchen 1 bis 4 und 8 bis 10 angegebenen Bedeutungen haben.

22. Schädlingsbekämpfungsmittel, welches als aktive Komponente eine Verbindung der Formel I gemäss einem der Ansprüche 1 bis 18 zusammen mit geeigneten Trägern und/oder anderen Zuschlagstoffen enthält.

23. Verwendung einer Verbindung der Formel I gemäss einem der Ansprüche 1 bis 18 zur Bekämpfung von Insekten und Vertretern der Ordnung Akarina.

24. Verwendung gemäss Anspruch 23 zur Bekämpfung larvaler Stadien pflanzenschädigender Insekten.

25. Verwendung gemäss Anspruch 24 zur Bekämpfung von Baumwoll-Schädlingen.

26. Verfahren zur Bekämpfung von Insekten und Vertretern der Ordnung Akarina, dadurch gekennzeichnet, dass man diese Schädlinge bzw. deren verschiedene Entwicklungsstadien oder deren Aufenthaltsort mit einer pestizid wirksamen Menge einer Verbindung der Formel I gemäss einem der Ansprüche 1 bis 18 oder mit einem Mittel enthaltend neben Zusatz- und Trägerstoffen eine pestizid wirksame Menge dieser Verbindung, in Kontakt bringt oder behandelt.